# EUROPEAN PATENT SPECIFICATION

(11) **EP 4 054 523 B1**
(45) Date of publication and mention of the grant of the patent: **06.08.2025**
(21) Application number: 20811756.4
(22) Date of filing: 02.11.2020
(51) Int. Cl.: A61K 9/00, A61K 47/24, A61K 47/36, A61P 3/02

(54) **ORALLY ADMINISTRABLE FILMS COMPRISING POORLY WATER SOLUBLE ACTIVE INGREDIENTS AND PREPARATION THEREOF**
ORAL VERABREICHBARE FILME MIT SCHWER WASSERLÖSLICHEN WIRKSTOFFEN UND IHRE HERSTELLUNG
FILMS ADMINISTRABLES PAR VOIE ORALE COMPRENANT DES PRINCIPES ACTIFS PEU SOLUBLES DANS L'EAU ET LEUR PRÉPARATION

(30) Priority: 04.11.2019 US 201962929963 P; 04.11.2019 US 201962929969 P; 04.11.2019 US 201962930053 P
(43) Date of publication of application: 14.09.2022
(73) Proprietor: Biota Ltd., Or Akiva 3061432 (IL)
(72) Inventor: HABER, Meir, 3061432 Or Akiva (IL); GABBAY, David, 4644018 Herzliya (IL); MASHAL, Revital, 3750410 Karkur (IL); SANCHIK, Michal, 4060000 Tel Mond (IL)
(74) Representative: Ter Meer Steinmeister & Partner
(86) International application number: PCT/IL2020/051137
(87) International publication number: WO 2021/090309

(56) References cited:
- WO-A1-2007/073346
- WO-A2-2007/125533
- JP-A- 2018 203 618

## Description

### FIELD

The present invention pertains to the orally administrable mucoadhesive films based on alginate polymer(s), with a high loading of poorly water-soluble bioactive material, as well as to the methods of manufacturing same.

### BACKGROUND

Oral administration of bioactive substances is commonly used in fields of pharmaceuticals, oral care products, and nutritional supplements. There are numerous bioactive materials. Such materials or substances, referred to in the pharmaceutical industry as active pharmaceutical ingredients (APIs) are incorporated in products such as dietary supplements, oral care products, and drugs, in order to provide improved health or prevent diseases, or otherwise benefit the wellbeing.

Bioactive substance are conventionally administered as products in a variety of dosage forms, e.g. in the form of syrups, tablets or capsules, however, the use of polymeric films, e.g. orally disintegrating films or mucoadhesive films, for the delivery of bioactive materials is also known in the field. Products comprising bioactive substances usually stabilize the substances both physically and chemically.

Orally administrable mucoadhesive film is a delivery system incorporating active substances in a polymeric matrix, which adheres to oral mucosal membranes. Upon placement of the film in the oral cavity and hydration of the film by saliva, the biologically active substance incorporated within the film is released from the matrix via diffusion and/or due to the erosion/disintegration or dissolution of the matrix containing the biologically active material. The bioactive material released from the matrix is sometimes cleared from the oral cavity by swallowing, but may also be absorbed through the mucous membranes in the oral cavities, thereby bypassing the hepatic clearance system.

It is known that the clinical efficacy of oral dosage forms of poorly water soluble substances, such as coenzyme Q10 (CoQ10) or lutein, is often limited by the low bioavailability of such bioactive substances (Sato, et. al., Biol. Pharm. Bull. 36(12) 2012-2017 (2013)).

CoQ10, also known as ubiquinol, or ubidecarenone, is a 1,4-benzoquinone, as described below, where Q refers to the quinone chemical group and 10 refers to the decaisoprenyl radical, e.g. the number of isoprenyl chemical subunits in its "tail". CoQ10 is a lipophilic vitamin-like fat-soluble substance, present in all respiring eukaryotic cells. CoQ10 is a yellow or orange solid having a melting point at 48-52 °C. CoQ10 is often present in products marketed as a dietary supplement, since reduced concentration of CoQ10 in a subject was found to be associated with several conditions, including cardiovascular diseases, neurological diseases, muscular dystrophy, and periodontal disease. Due to its size and structure, CoQ10 is practically insoluble in water, exhibiting an extremely low water solubility of, for example, between 2-3 parts per million (ppm) (i.e., 2-3 mg/L). The most commonly used methodology today with regards to CoQ10 delivery systems includes a gel capsule comprising the CoQ10 therein. Most of the CoQ10 products known today are formulated as swallowed soft-gel capsules marketed at recommended daily dosages ranging from 60 to 400 milligrams, or higher. The challenges related to consumers' adherence to conventional CoQ10 soft gel and similar swallowed products are described in US Patent 9,775,332.

Numerous delivery strategies are known in the art for assisting in overcoming the limitations of oral dosage forms of poorly soluble active materials, which include *inter alia,* micronized particles for increasing the surface area and improving the dissolution rate of the active materials, nanoparticulate systems, complexation to polymers and inclusion complexes, gelation, incorporating the active material within a soft gel capsules in dissolved form, powder amorphization, etc.

Another approach for achieving an improved administration of poorly bioavailable active materials is via mucoadhesive films. Common components allowing mucoadhesion are, among others, alginate-based materials. Alginates are negatively charged, natural polysaccharides comprising guluronate and mannuronate structural units, which can be arranged randomly, in blocks, or alternatingly. Alginates pertain to the group of generally recognized as safe (GRAS) materials by regulatory authorities. The use of alginates, including blending alginates with other natural or synthetic polymers, for producing oral mucoadhesive films has been described in some previous publications. For example, US Patent 8,840,935 / WO 2007/125533 provides details regarding the features of such films, including aspects of alginate viscosity grade, film thickness, oral residence times, organoleptic properties, mechanical properties, and challenges related to films' preparation process. Further elaborations on the oral films properties related to mucoadhesion and oral residence time are disclosed in US Patent 10,285,910.

Films comprising CoQ10 and alginate have been demonstrated also in Japanese patent application JP 2018203618. In the publication, films were made utilizing a relatively high concentration of film-forming emulsifier octenyl succinic acid starch (up to 1:1 ratio with the active ingredient). The document discloses and exemplifies a two-step process including forming a CoQ10-succinylated starch aqueous emulsion by high-shear homogenization, and mixing the formed emulsion with alginate solution to obtain a casting mass to the films. A further publication US Patent 9,655,849 discloses the improvements in the bioavailability and/or solubility of coenzyme Q10, providing methods for preparing particulate compositions including coenzyme Q10, that generally comprise dispersing and/or dissolving the coenzyme Q10 in a suitable solvent, and combining the coenzyme Q10 mixture with an encapsulating agent. Additionally, US patent application US2019/0269649 discloses liposomal and specialty formulations intended to resolve the challenges of incorporation oily plant extracts into oral film strips, indicating that direct incorporation of such oily extracts typically results in sticky mixtures which do not form processable film having desirable properties, such as being dry to touch, easily handled and packaged, and/or easily consumed.

Incorporation of poorly water soluble materials into a hydrophilic matrix is often a non-straightforward task. The challenge is stemming from the fact that the concentration of such bioactive substances may be limited as it is forced into close contact with hydrophilic polymer matrices. Additionally, the incorporation of lipophilic substances into hydrophilic matrix, especially a solid matrix, may require the use of large quantities of emulsifying and/or solubilizing agents in order to maintain the thermodynamic stability of said film, prevent blooming (i.e. leakage) of the lipophilic substance from the matrix. The emulsifier concentration in the matrix is usually dependent on its type, and can even be twice the concentration of the bioactive material present in said film. The loading of poorly soluble substance in a hydrophilic matrix is thus rather limited. This limitation becomes even more pronounced if a large amount of an active material, which may be in the form of an oily liquid, are to be processed with a hydrophilic polymer into a matrix due phase separation tendency, e.g. during the preparation process of the composition (separation of the oil-in-water emulsion) or, later, during the storage period of the obtained films. For example, US Patent 8,758,803 discloses film-shaped preparations, rapidly disintegrating on contact with saliva or other aqueous liquids, which are capable of stably containing a large amount of at least one oily substance; it is worthwhile noting that the incorporated oily substance has been found to exude from films prepared with alginate.

Similarly, US patent 8,759,282 / WO 2007/073346 discloses films of soluble APIs, or APIs with pH-sensitive solubility that are slightly soluble in deionized water, such as ibuprofen (21 mg/L at 20 °C), in a selected alginate without use of any emulsifier.

Additionally, PCT patent application WO2019/219773 discloses similar alginate films with cannabinoids, e.g. 5 to 20 mg cannabidiol (CBD) per dosage unit as described therein. CBD aqueous solubility is 12.6 mg/L, thus CBD is considered a poorly water-soluble or lipophilic drug. CBD was incorporated into alginate films with a large amount of surfactants / emulsion base, with organic co-solvents, or in pH above 12 wherein CBD is soluble in water.

There is still a need in the art to provide mucoadhesive films comprising poorly soluble bioactive ingredients in a sufficiently high loading. There is a specific need to provide high-loading natural bioactive ingredients mucoadhesive films comprising only of natural ingredients, e.g. CoQ10. The films should be sufficiently uniform, readily manufactured, without cracks, preferably chemically stable, and adhere and reside at oral mucosal membrane, should not exhibit exudation, be palatable, and have an acceptably long shelf life.

### SUMMARY OF THE INVENTION

The present invention provides a mucoadhesive orally administrable alginate film as defined in claim 9. Such mucoadhesive films are often difficult to achieve. Due to the poor water solubility of the active material, it cannot be readily dissolved together with the film-forming agent(s).

It has now been unexpectedly found that films of acceptable quality can be obtained by homogenizing a dispersion of poorly soluble bioactive material in an aqueous alginate solution (e.g. as opposed to conventional approach where the solution of the film-forming polymer is added after the active agent has been homogenized at low viscosity). In one aspect, provided herein is a method of manufacturing the mucoadhesive film as defined in claim 1. The mucoadhesive film comprises at least one alginate(s) as the major film forming polymer, as elaborated in greater detail below. The method comprises homogenizing an aqueous solution of the alginate(s) with a poorly water-soluble bioactive material and an emulsifier, to provide a mass suitable for casting. The homogenizing is achieved under high weight ratios between the bioactive compound and the alginate(s) between 1:2.5 and 2.5:1, e.g. between 1:2 and 2.5:1, or e.g. between 1:1 and 2.5:1. As the result of homogenizing step, obtained thereby a mass suitable for casting, which can be further processed into the film, e.g. by casting and drying.

Preferably, prior to homogenizing step, the poorly water-soluble bioactive material may be combined with the emulsifier, and then the resultant mixture may be combined with the alginate solution.

Alternatively, prior to homogenizing step, the alginate solution may be combined with the emulsifier, and then the resultant mixture may be combined with the poorly water-soluble bioactive material.

Further alternatively, prior to homogenizing step, the poorly water-soluble bioactive material may be mixed , e.g. in a molten form, for example when the active agent is CoQ10, with an emulsifier, and the resultant mixture may be combined with an adsorbing powder; the resultant mixture may be further ground, and then combined with the alginate solution.

The homogenized mixture, i.e. the mass suitable for casting, may be cast onto a suitable liner, particularly with the wet thickness of over 1 mm, e.g. between 1 and 3 mm. Preferably, the casting may be performed with a slit-die apparatus, at any suitable temperature, but may sometimes be performed at a temperature between 50°C and 85°C.

Described herein is an orally administrable mucoadhesive film, obtainable according to the methods as essentially described herein.

Additionally, provided herein an orally administrable mucoadhesive film comprising at least one poorly water-soluble bioactive material, an emulsifier, and an alginate. The alginate is the major film forming polymer in the film. The film is characterized by the high loading of the poorly water-soluble bioactive material in the film, which is between 15 wt% and 45 %wt, e.g., 20-30 wt% of the dry weight of said film. Alternatively or additionally, the high loading of the poorly water-soluble bioactive material is such that the dose thereof is between 5 and 20 milligrams in a square centimeter of the film, with dry film thickness varying in the range from 100 to 400 µm, and wherein the weight ratio between the bioactive material and the alginate is from 1:2.5 to 2.5:1..

In the orally administrable mucoadhesive film according to some preferred embodiments of the invention, the film comprises at least one poorly water-soluble bioactive material, at least one emulsifier, and an alginate as major film forming polymer, the weight ratio between said bioactive material and said alginate is from 1:2.5 to 2.5:1, and preferably between 1:2 and 2.5:1, e.g. 1:1 and 2.5:1. The concentration of the emulsifier is usually less than 1/5 of the concentration of the poorly water-soluble bioactive material, e.g. between 1/10 and 1/100, preferably between 1/50 and 1/60. The emulsifier may be selected from the group consisting of phospholipids, sorbitan fatty acid esters, ethoxylated sorbitan esters, ethoxylated fatty acid esters or fatty alcohol ethers, sucrose fatty acid esters, and combinations thereof. The emulsifier is preferably a phospholipid, e.g. lecithin.

According to some preferred embodiments of the invention the poorly water-soluble bioactive material usually has aqueous solubility below 10 mg/L at 20 °C, e.g. between 0.1 and 8 mg/L at 20 °C, or between 1 and 5 mg/L; and it may also be essentially insoluble in the film, as elaborated below. The poorly water-soluble bioactive material may usually be selected from the group consisting of vitamins or vitamin-like substances that are naturally present in the human body, carotenes, terpenes, flavonoids, cannabinoids, natural oils, and plant extracts. In some preferred embodiments, the poorly water-soluble bioactive material is coenzyme Q10, preferably in a concentration of between 10 and 50 wt%, e.g. between 15 wt% and 45 wt%, based on the dry weight of the film. In some particularly preferred embodiments, the film comprises coenzyme Q10, alginate, and lecithin. The mucoadhesive film may comprise between 10 and 45 wt% of the dry weight of the film of an alginate (e.g. between 25 wt% and 45 wt%); between 10 and 50 wt% of the dry weight of the film of coenzyme Q10 (e.g. between 15 wt% and 45 wt%, more specifically between 15 wt% and 25 wt%), and between 0.01 and 5.0 wt% of the dry weight of the film lecithin (e.g. between 0.05 wt% and 0.2 wt%). The film may also further comprise one or more of film-forming polymers other than alginate, plasticizers, flavors and/or fillers, up to 100 %wt of the formulation.

### DETAILED DESCRIPTION

Generally, as described in greater detail below, the films are dry polymeric compositions formed by the polymer matrix-former (i.e. film-former), with additional components, such as, *inter alia,* plasticizers, taste-masking agents, and of course, bioactive materials, dissolved or dispersed in the film. The films generally are produced by providing a solution of the film-forming polymer(s) (although some polymers may form so-called latex-type films by casting from dispersions at a temperature to enable fusion of the particles into a film; alginate films are usually produced from solution), and dissolving or dispersing therein the additional components, to provide a precursor composition which is suitable for casting, i.e. casting solution, sometimes referred to as "wet film" (when cast onto a liner but not yet dried) or "casting solution ", "active mass", or "mass suitable for casting" or "mass", as used interchangeably herein. The casting solution contains all the components of the film in the ratios as needed (and in context of the present invention, as defined below), together with solvent(s) that evaporate during the drying of the film. It therefore usually has the composition of the dry film, with the ratios between the components as described in great detail below, and in addition comprises a solvent, e.g. an essentially aqueous medium, which eventually evaporates during the drying step (apart from residual moisture). The casting solution is then applied onto a support (i.e. substrate, such as a liner as elaborated below) and dried to furnish a finished, i.e. dry film, which can then be further processed, e.g. laminated, rolled, cut to a shape as needed, and/or packed into unit doses. Therefore, the amounts of the components in the composition are selected to meet the requirements of these two separate aspects: the wet film and the resulting dry film.

The amount in the dry film, frequently referred to as "dry weight", unless explicitly mentioned otherwise, is usually presented in the hypothetically dried film, which in reality usually contains some residual moisture. For alginate films the residual moisture content may be rather high, e.g. between 5 and 15 weight percent. The residual moisture can be readily determined by drying at elevated temperature to a constant weight, e.g. at 110°C, and is frequently referred to as loss-on-drying (LOD). Therefore, the amounts of the components in the composition are usually given without reference to LOD.

The term "major film forming polymer" as used herein usually refers to alginate or to a mixture of more than one alginate, and to the fact that the total concentration of alginate(s) by weight in the film is greater than that of any other film-forming polymer that may be included in the film. Alginate thus forms the polymeric film matrix. The ratio of alginate to all film-forming agents may also be over 1: 1, i.e. with alginate being at least 50% of the total film-forming agents. Preferably, alginate is over 60% of film-forming agents, and more preferably over 70%. In some embodiments, alginate is the sole film-forming agent, i.e. the film-forming composition in the film consists essentially of alginate. The film may further comprise additional excipients, e.g. plasticizers, sweeteners, preservatives, buffering agents, fillers (bulking agents), co-film-formers, co-solvents, stabilizers, and flavors and colorants.

It has now been unexpectedly found that films of acceptable quality and processability, i.e. films being uniform throughout the cast in terms of thickness and content, being flexible, and mechanically strong, can be obtained by homogenizing a dispersion of poorly soluble bioactive material in an alginate solution. It is customary to avoid homogenization of solutions containing high amount of polymers, and as a rule, having a high viscosity. Such homogenization usually is not a problem-free process, causing overheating of the equipment, requiring meticulous control over the mass and heat transfer in the reactor, to avoid focal heating around and/or inside the parts of some of homogenizers, and introduces large quantities of poorly removable air bubbles. However, if a relatively high amount of poorly soluble bioactive material is homogenized with an alginate, particularly in presence of a low amount of an emulsifier, with a specific weight ratio of the bioactive material to alginate, and optionally, to the emulsifier as generally described herein, it is possible to conduct the homogenization of the mixture to furnish an active mass suitable for casting into a film. The air bubbles entrapped in thus produced active mass can be readily removed by standard degassing techniques. Moreover, conducting the process in a conventional way results in a much more brittle film with cracks, which decrease substantially the useful film area to be cut into dosage units.

The uniformity of the obtained film is of paramount importance for the further processing into final dosage units. The uniformity may usually be expressed in terms of weight variation per a unit area of the film. The variation is usually the relative standard deviation, expressed in percentage. The measure is obtained by accurately weighing units of film normalized by their area, obtaining a mean weight and a standard deviation of the weights in the sample, and dividing the standard deviation by the mean. Usually, the standard deviation of the mean weight per standard area is lower than 20%, preferably lower than 15%, further preferably lower than 12%, or lower than 10%, or lower than 8%, or lower than 7%, or lower than 6%, or lower than 5%. The uniformity may also be expressed as the relative standard deviation of mean content of the poorly water-soluble bioactive material per unit of area. Additionally, the uniformity may be expressed by the relative standard deviation of mean thickness of the obtained film. It is also readily appreciated that the uniformity implies that the film as cast and dried, is usually free of powder aggregates, and that the film covers the whole area of the cast, i.e. without segregation which is known to occur under certain conditions, e.g. due to incompatibility of the surface tension between the active mass and the liner, causing the active mass to assemble into islets on the liner, unsuitable drying regimen, etc.

The obtained film is usually sufficiently flexible and mechanically strong to withstand usual handling of the film (sometimes referred to as "processable"). The properties of the film include the flexibility and the strength. The flexible film can be readily deformed at least several times without any major failure (cracking), and a strong film does not tear upon a standard handling deformation, e.g. upon detachment from the substrate liner. One way to evaluate the film for acceptability towards processing is described in the Examples section, under "folding endurance" test. The processable films having an appropriate flexibility and mechanical strength can be readily cut into unit dosages of desired shape and size, without creating cracks in the cut dosage unit, or a propagating crack in the film being cut.

The films according to the invention are mucoadhesive, i.e. they adhere well to the mucosa of the oral cavity upon contact therewith and an application of slight pressure. The films also usually exhibit an acceptable oral residence time, as described in greater detail below.

The process according to the invention is characterized by homogenizing the aqueous solution of alginate, which contains the poorly water-soluble bioactive material, e.g. in presence of an emulsifier. When the weight ratio between the poorly-water soluble bioactive material and alginate is high, e.g. from 1:2.5 to 2.5:1, and particularly when the amount of the emulsifier is less than 1/5 of the concentration of the poorly water-soluble bioactive material, the homogenization may be readily achieved, e.g. without excessive means for degassing.

The homogenization may be performed using by a variety of methods known in the art. The homogenizing step is performed using high-shear mixers, e.g. of rotor-stator type homogenizers, ultra-turrax type homogenizers, or mechanical mixers equipped with high-shear impellers of a suitable structure, according to the vessel used. The specific impeller structure, and operative velocities conductive to particular shearing intensities will be dependent on the apparatus used, but may vary depending on the viscosity and the composition of the fluid, and other parameters. The typical velocities of a shear type impeller may be at over 1,000 revolutions per minute (rpm), preferably at or over about 1,500 rpm, and further preferably at or even over 5,000 rpm. The homogenization may be performed continuously or intermittently, at the same or different, e.g. escalating, velocity. Without being bound by a particular theory, it is hypothesized that in presence of high amount of poorly water-soluble bioactive material and particularly of a relatively low amount of an emulsifier, the alginate is the one that stabilizes the poorly water-soluble bioactive material in the mass and later, during and after the drying stage, thus producing a uniform homogenous film in which poorly water-soluble bioactive material being uniformly dispersed in the film matrix, to allow sufficient flexibility and mechanical strength of the film.

Degassing of the active mass is aimed to allow evacuation of the air bubbles from the solution. These bubbles, however small in size, will inevitably grow during the drying of the film, due to the evaporation interface that they present, and considering the solidifying film components around, impeding evacuation of the bubble from the cast wet film. These bubbles create weak spots, initiate and propagate cracks, increase the weight and thickness variability, cause delamination of the film, and overall decrease the film quality. The degassing is usually carried out by a variety of methods, including the addition of anti-foaming agents, centrifugation, exposure to (high) vacuum, and other techniques. The most common technique, however, is a slow stirring of the solution under very low shear rate, preferably under reduced pressure and slightly elevated temperature, in a closed system, to avoid the loss of the solvent. By the processes according to the invention, it has been surprisingly found that the simple degassing technique may be sufficient to release all the entrapped bubbles in the highly viscous active mass mixture.

The homogenizing step is therefore a process performed in an essentially aqueous medium. The term "essentially aqueous medium" indicates that water, e.g. deionized water of pharmaceutical or food quality, is the major solvent of the mixture undergoing the homogenizing step. The essentially aqueous medium may further comprise non-aqueous liquid components, such as plasticizers, oils, flavors, or organic co-solvents, provided that the amounts thereof are not sufficient to dissolve the poorly water-soluble bioactive material to any appreciable extent, e.g. the undissolved fraction of the poorly soluble bioactive material is more than 85%, or preferably more than 99%, in the essentially aqueous medium. The essentially aqueous medium usually comprises water between 75 %wt and 100 %wt, and preferably comprises above 80 %wt of water, above 85 %wt, above 90 %wt, or most preferably above 99 %wt of water, of the total weight of the solvent(s).

The homogenizing step is thus carried out on aqueous alginate(s) solution, and poorly water-soluble bioactive material(s), and emulsifier(s), but the mixture for the homogenizing step may also comprise ingredients including additional film-forming polymers (provided that alginate is the major film-forming polymer), plasticizers, fillers, flavors, taste-masking agents, and/or any further component that may be present in the film, as described below.

The homogenizing step and, separately, degassing step, may each be performed at reduced pressure. The use of reduced pressure allows less air to be entrapped in the solution, and accelerate the evacuation of the entrapped air. Usually, the reduced pressure values suitable for the homogenizing and/or degassing steps is readily determined according to the equipment used, the working temperature, the use of condensers, etc. Preferably, when degassing and/or homogenizing is performed under reduced pressure, the process would complementarily comprises cooling and condensing the vapors of the alginate(s) solution or homogenized active mass, and feeding the condensate back into the alginate solution or homogenized active mass.

The mixture for the homogenizing step may be prepared by at least three alternative processes. It has now been unexpectedly found that the poorly soluble bioactive materials, particularly such as having a relatively low melting point, e.g. below 80°C, can be readily combined with an emulsifier, and even when low amount of the emulsifier is used as described above, the poorly soluble bioactive material can be successfully and adequately dispersed in the aqueous media comprising alginates. One example of such bioactive material is CoQ10. Moreover, the emulsifier may be added to the poorly soluble bioactive material or to the alginate solution, and nevertheless the poorly soluble bioactive material can be adequately dispersed in the casting solution.

Thus, in one alternative, the poorly water-soluble bioactive material is combined with the emulsifier, and optionally with further lipophilic components, e.g. an antioxidant or a preservative. This combining of the poorly water-soluble bioactive material with the emulsifier may comprise mixing, melting, or otherwise creating a uniform mixture of these materials. Separately, the alginate is combined with water and other components of the essentially aqueous medium, and mixed until dissolution. The time required to dissolve the alginate in the essentially aqueous medium is usually dependent on the primary dispersion of the powder and wetting thereof; if proper care is taken to avoid lumping, the dissolution times required to completely dissolve alginate powder in water range between 15 and 120 minutes. The dissolution of the alginate may be performed at any convenient temperature, but it may be advantageous to perform the dissolving the alginate at elevated temperature, e.g. between 50 °C and 90°C, preferably between 50°C and 60°C. The combining of poorly water-soluble bioactive material with other components as described above may also be performed at elevated temperatures, particularly and advantageously, when the poorly water-soluble bioactive material has a melting point in the same range, like coenzyme Q10.

Alternatively, the essentially aqueous medium may be combined consecutively or concomitantly with alginate and an emulsifier, and mixed until dissolution, as described above. To the obtained mixture, the poorly water-soluble bioactive material may be provided, to furnish the mixture ready for the homogenizing step.

Additionally, the poorly water-soluble bioactive material may be combined with an emulsifier, and then combined, e.g. thoroughly mixed, with an adsorbing powder. The obtained mixture may then be subjected to grinding, to reduce the particle size of the poorly water-soluble bioactive material. Advantageously, the adsorbing powder may be the powder of any further components of the film, e.g. the solid plasticizer (such as xylitol), a filler, a further film-forming polymer, and the like.

The ready active mass, downstream to the homogenizing step, is then cast onto a substrate to furnish a wet film, i.e. the processes of the invention comprise a casting step. The wet film formed as described above is dried, e.g. utilizing a circulating hot air oven. The casting process may be performed using a variety of methods. In one preferred alternative, the casting is performed by a slit-die assembly. The slit-die assembly is an apparatus characterized by a gap-forming plates, wherethrough the active mass is forced under positive pressure, or allowed to flow freely, onto the substrate, which is usually moving at a predetermined speed relative the static slit-die assembly. The slit-die assembly is usually in liquid communication with an active mass tank, wherein the active mass is stored at controlled temperature until being cast onto the liner. The slit-die apparatus is usually connected to the active mass tank via a pump, usually a peristaltic pump, a piston pump, or rotor-stator pump, allowing to control the feeding rate of the active mass. The slit-die apparatus plates may be optionally heated to a controlled temperature, to maintain or increase the temperature of the active mass. The active mass may be also be circulated through the slit-die apparatus at a predetermined rate, e.g. to avoid sedimentation of undissolved components and clogging of the slit, with only the required amount being allowed to flow out of the die. A further apparatus that can be used for the casting is a draw blade. The draw blade is a blade placed over the substrate at a predetermined gap. The blade or the substrate, or sometimes both, are moved one relative to the other, and the casting solution is placed before the draw blade in the forward direction, so that the active mass is allowed to flow in the gap of the draw blade and the substrate. The active mass may be supplied to the position right before the draw blade, e.g. from the active mass tank via a peristaltic pump. Depending on the width of the draw blade, more than one supply outlets may be used to supply the active mass to the draw blade, to ensure a uniform coating. Casting can be also performed by further methods commonly used in the field such as gravure, printing, and the like.

The temperature of the casting step is usually determined by the temperature of the supplied active mass, and can be additionally controlled, e.g. by heated slit-die assembly. Whereas the casting process may be performed at ambient temperature, it has been found that conducting the process at elevated temperature, e.g. between 40 °C and 90°C, preferably between 50°C and 65°C, may be particularly advantageous.

The casting technique is intended to allow a uniform film thickness and uniform bioactive material distribution within the film. One of the main properties that commonly affect the film casting process and the resulted film obtained by the process is the viscosity of the casting solution prior to its casting on the substrate. Typical industrial process casting is performed at ambient temperature, and with the active mass having a viscosity range of 4,000 cP (mPa*s) to less than 7,000 cP (mPa*s), but as demonstrated in the appended Examples, it has now been surprisingly found that utilizing the mass as described herein it is possible to perform continuous slit-die casting at a high viscosity, e.g. at over 12,000 cP, and at wet thickness over 1,500 microns and even over 2,000 microns. The wet thickness of the wet film is usually determined by the gap of casting instrument, e.g. the slit-die, or the draw blade or knife. It is customary in the art that the wet thickness should not exceed 1000 micrometers, and should preferably be significantly less. However, providing the casting solution as defined herein, it was possible to cast wet films with thickness over 1000 microns, e.g. between 1.0 and 3.0 mm, e.g. between 1250 and 3000 µm, or between 1250 and 1800 µm. The viscosity of the active mass to be used in casting step is usually between 10 Pa*s and 40 Pa*s at 25°C, or between 5 Pa*s and 35 Pa*s at a temperature between 50°C and 85°C.

Typical industrial process employs casting onto a roll of release liner, e.g. thin (usually less than 100 microns) PET film, drawn at a constant linear speed through a drying chamber, and at the exit side it is re-rolled with the dried formed film on top of the release liner. The commonly used substrates are plastic sheets such as polyethylene terephthalate (PET), siliconized PET, native or siliconized biaxially oriented PET (known as Mylar^{™}), polytetrafluoroethylene (Teflon^{™}), polyethylene, or polypropylene, and sometimes stainless steel may be used for tray drying.

An exemplary industrial continuous coating and drying machine which is based on slit ultra-precise coating knife system, is a machine of OPTIMAGS^{™} MBCD series. These machines enable precise coating and higher film uniformity than the one obtained by commonly applied in aqueous solvent evaporation film forming process. It is worthwhile noting, that according to OPTIMAGS guidelines for oral films maximum active mass viscosities is 7,000 cP, with casting is preferably performed at ambient temperature, and the maximum wet thickness of the coated active mass is up to 1000 µm. It should be noted that industrial manufacturing of films of the invention by performing slit-die casting at commonly unacceptably high viscosity and high wet thickness may be required in order to obtain the intended high dosage, low dry thickness variability, and high content uniformity of the films of the invention, with the viscosity and the wet thickness being much higher than industrially practiced.

Additionally, the process may further comprise a step of cutting the formed film into a desired shape, e.g. in a strip form, and packing in an appropriate single or multi-unit packaging, such as sachets or cassettes. Single unit sachets packaging are preferably aluminum foil laminate sealed by heat.

In certain specific embodiments, the process according to the invention comprises i) providing a casting solution with composition as generally described above, the casting solution comprising a solution of alginate and an emulsifier in an aqueous medium, and further comprising a bioactive material which is essentially insoluble in the casting solution, ii) casting the casting solution onto a substrate to furnish a wet film, and iii) drying the wet film to desired residual moisture to obtain dried film (i.e. dry film).

In further specific embodiments, the process according to the invention comprises the following steps:
i) providing the poorly soluble bioactive material; ii) providing an alginate aqueous solution, wherein the alginate solution comprises an emulsifier; iii) mixing the poorly soluble bioactive material of step (i) together with the alginate solution of step (ii), and iv) homogenizing the obtained mixture to furnish casting solution; (v) casting the casting solution obtained in step (iv) on a surface of a substrate to obtain a wet film; and vi) drying the wet film to obtain a dry film, which is a stable, homogeneous, solid mucoadhesive film comprising the poorly soluble bioactive material for oral administration.

In an alternative specific embodiments, the process according to the invention comprises the steps of: i) mixing said poorly soluble bioactive material with an emulsifier in a non-aqueous environment; ii) providing an alginate aqueous solution; iii) mixing mixture prepared in step (i) together with the alginate solution of step (ii) and iv) homogenizing the obtained mixture to furnish casting solution; (v) casting the casting solution obtained in step (iv) on a surface of a substrate to obtain a wet film; and vi) drying the wet film to obtain a dry film, which is a stable, homogeneous, solid film comprising said poorly soluble bioactive material for oral administration.

Similarly, the alginate solution may further comprise components buffering agents, chelating agents, plasticizers, antioxidants, minerals, bulking agents, co-film forming polymers, stabilizers, flavors, and colors, as generally described above. These materials may be added during the preparation process of the film to either the aqueous alginate solution, to the poorly soluble bioactive material or to their mixture.

The poorly soluble bioactive material may be added to the alginate solution of step. The homogenization process may then be carried out utilizing methods known in the art, such as high pressure piston homogenizer or shear type, e.g. high-speed ultra-turrax disperser. Preferably, the homogenization of step is performed utilizing vigorous stirring at high shear rate in order to emulsify the mixture, possibly utilizing an ultra-turrax type disperser.

In some embodiments, the poorly soluble bioactive material may be ground prior to its addition to the alginate solution, e.g. with additional solid components of the film. Without being bound to theory or mechanism of action, it is believed that the grinding of the poorly soluble bioactive material at this stage may provide a more homogeneous dispersion of the active material within the active mass and the film. Such separate step may enable better control of particle size and may prevent sticking and loss of molten materials to the surface of vessels.

In a further aspect, provided herein an orally administrable mucoadhesive film, prepared according to a method as described herein.

According to the principles of the invention, at least one bioactive material utilized in the film of the invention is a poorly water-soluble bioactive material. The term "poorly soluble bioactive material" or "poorly water-soluble bioactive material", as used interchangeably herein with the terms "drug", "active agent", "active pharmaceutical ingredient", "biologically active", and the like, in conjunction with the term "poorly soluble" or "poorly water-soluble", refer to a bioactive material with the solubility of below 0.01 g/L (i.e. 10 ppm), further preferably below 0.001 g/L (i.e. 1 ppm).

The poorly soluble bioactive agent may also be present in the film in an undissolved form, i.e. the bioactive material is essentially insoluble in the film. Therefore, the bioactive material that is essentially insoluble in the film is thus not only poorly soluble in reference to aqueous solutions, but also insoluble in the wet film or active mass. The amount of water in the wet film, as described in greater detail below, may *inter alia* be determined based on the desired final viscosity, e.g. between 7 and 40 Pa*s, or between 10 and 40 Pa*s, as described herein, and as measured according to the methods in the Examples' section. The bioactive material that is essentially insoluble in the film is thus present largely in undissolved form in a wet film comprising alginate film former and all other excipients, in a sufficient amount of water and/or co-solvents producing viscosity of between 7 and 40 Pa*s at 20 °C. Therefore, even when the film composition or the wet film comprise various co-solvents, surfactants, and/or oils, the bioactive material is not soluble in solvents therein to any significant extent, as described above.

The term "biologically active" as used interchangeably herein with the terms "drug", "active agent", "active pharmaceutical ingredient", "biologically active", and the like, refers to a substance which is of a natural or synthetic or semi-synthetic origin used to alleviate or treat or prevent diseases or health disorders such as migraine, multiple sclerosis, Parkinson's or Alzheimer's diseases, depression, dementia, anxiety, insomnia, fatigue, etc., or known to have a positive or negative influence on at least some human biological systems such as decreasing or increasing essential nutrients levels, *inter alia,* intended to preventing or restoring deficiencies. The term also refers to natural products or essential nutrients and refers, *inter alia,* to a material of natural, or synthetic or semi-synthetic origin, having the chemical structure identical to the natural product, or a modified natural product. The term may refer to a material which may be a mineral, one or more of vitamins and pro-vitamins or vitamin analogs or vitamin-like substances, omega 3 fatty acids, antioxidants, CoQ10, cannabinoids, terpenes, flavonoids, antifungal agents, plant extracts, steroids, phytosterols, polyphenols and poorly soluble phenols, carotenoids such as lutein, and phosphatidyl serine. A particularly preferred example of poorly water soluble bioactive substance is CoQ10. As described in greater detail below, CoQ10 exemplifies insights to the challenges of incorporating water insoluble bioactive substance in oral alginate films in high loading. Further preferred examples of the poorly soluble bioactive materials include omega-3 fatty acid, vitamin E, turmeric water-insoluble extract, lecithin, and lutein.

It has now been surprisingly found that utilizing the processes as described herein a relatively low concentration of an emulsifier is sufficient or needed, to provide an advantageous effect contributing to the stabilization of a poorly soluble drug, e.g. CoQ10, in the hydrophilic alginate matrix. The amount of the emulsifier(s) may be lower than 1/5 of the amount of the drug, and preferably is less than 1/10, or 1/20, or 1/50 of the amount of the drug, and down to 1/100 of the amount of the drug. The amount of the emulsifier is thus preferably between 1/20 and 1/100 of the amount of the drug, and currently particularly preferably between 1/50 and 1/60 of the amount of the poorly water-soluble bioactive material. Thus a higher loading of the drug could be achieved, with a weight percent ratio between drug and the alginate in the film of the invention being higher than 1:2.5, e.g. higher than 1:2, higher than 1:1. In some embodiments, the weight ratio of the drug to alginate is between about 1:2.5 to about 2.5:1, e.g. between 1:2 to 2.5:1, e.g. between 1:1 and 2.5:1. Preferably, the weight ratio of bioactive material to alginate is between about 1:2 to about 2.5:1.

Therefore, the loading of the bioactive material, i.e., the total amount of the bioactive material in the dry film as expressed in the weight percent of the film, is above about 5 wt%, preferably above 10 wt%, e.g. between 10 wt% and 50 wt%, or between 15 wt% and 45 wt%. An orally administrable mucoadhesive film with the concentration of poorly water-soluble bioactive material in the film over 10 wt% of the dry weight of the film therefore forms a further aspect of the present invention.

The amount of the poorly water-soluble bioactive material in the film that may be reached according to the invention, is between 5 and 30 mg of the drug per one square cm of the dry film with dry film thickness varying in the range from 50 to 600 µm, preferably from 100 to 400 µm, e.g., between 7 and 15 mg. Preferably, the orally administrable mucoadhesive film contains the amount of poorly water-soluble bioactive material in the film between 5 and 20 mg per cm² of the dry weight of the film therefore forms a further aspect of the present invention.

The thickness of the film is usually governed by the thickness of the wet film cast onto the substrate and the drying conditions, and of course by the formulation. For example, when a high drug dose is required, a film may be prepared of a higher thickness and/or applied in larger pieces, but this may in turn impair the oral residence time and/or adhesion to the roof of the mouth, not to mention the patients' compliance. The dry thickness of the film of the invention, i.e. the thickness of the film obtained after drying wet film to a target moisture content value, may usually be in a range from about 0.030 mm to about 3.0 mm, more preferably from about 0.10 mm to about 1.0 mm.

Additionally, according to the principles of the present invention, the emulsifier content which is required for achieving the advantageous stabilization of said bioactive material within the alginate matrix is lower than the bioactive material content in the film. The amount of the emulsifier is usually less than 1/5 of the amount of the bioactive material, preferably less than 1/10 of the amount, less than 1/25 of the amount, or even less than 1/50 of the amount of the bioactive material, and down to 1/100 of the amount of the drug. The amount of the emulsifier is thus preferably between 1/20 and 1/100 of the amount of the drug, and currently particularly preferably between 1/50 and 1/60 of the amount of the poorly water-soluble bioactive material.

The emulsifier that may be utilized in the relatively low concentration according to the invention may be any suitable emulsifier compatible with the poorly soluble bioactive material, e.g. cationic emulsifier, anionic emulsifier, zwitterionic emulsifier, or a non-ionic emulsifier.

Preferably, the emulsifier utilized as described above is selected phospholipids, such as phophatidylethanolamine, phosphatidylinositol, phosphatidylserine, lecithin and their derivatives. In a currently preferred embodiment, the emulsifier is lecithin. Lecithin may be a solid lecithin, or, preferably a liquid lecithin.

The emulsifier may also be selected from the group consisting of phospholipids; bile salts; sorbitan fatty acid esters; ethoxylated sorbitan esters; sucrose fatty acid esters; ethoxylated fatty acid esters or fatty alcohol ethers; and combinations thereof.. Suitable emulsifiers also include PEG-fatty acid glycerides, e.g. polyethylene glycol 12-hydroxy stearate. Sorbitan fatty acid esters are also known under the name Span^{™}, e.g. sorbitan monolaurate (Span^{™} 20), sorbitan monopalmitate (Span^{™} 40), sorbitane monostearate (Span^{™} 60), sorbitane tristearate (Span^{™} 65), sorbitane monooleate (Span^{™} 80), sorbitane trioleate (Span^{™} 85), or sorbitane sesquioleate (Span^{™} 83). Ethoxylated sorbitan esters are also known under the name Tween^{™}, e.g. polyoxyethylene (20) sorbitan monolaurate (Tween^{™} 20), polyoxyethylene (20) sorbitan monopalmitate (Tween^{™} 40), polyoxyethylene (20) sorbitan monostearate (Tween^{™} 60), and polyoxyethylene (20) sorbitan monooleate (Tween^{™} 80). Sucrose fatty acid esters are also known under the name Sisterna^{™} or Ryoto^{™} materials, e.g., sucrose stearates (Sisterna SP50, Sisterna SP70), or sucrose palmitate (Sisterna PS750), or ethoxylated fatty acid esters and ethoxylated fatty alcohol ethers. It should be noted that the use of lecithin is also highly advantageous as it is a natural plant-based, non-allergenic material, which may serve as a single safe emulsifier in the film delivery system of the invention.

The alginate utilized in the invention as a major film forming agent may be of a single type or a mixture of several types. Alginate may originate from brown seaweeds, microbial fermentation, or a synthetic source, and may be further synthetically modified, e.g., propylene glycol alginate. In a currently preferred embodiment, the alginate of the invention is sodium alginate. The alginate utilized in the invention may be of one single grade or a combination of several different grades. It is to be understood that the grade of alginate is normally classified by its apparent viscosity, and the utilized alginate grade or blend of grades may be of any suitable nominal viscosity. Preferred alginate of the invention is a blend of more than one grade, most preferably blend of low and very low viscosity grades.

**Table 1**

| Alginate | Type | Viscosity of 1 wt% aqueous solution (mPa*s) |
|---|---|---|
| Protanal^{®} LFR5/60 | Very low viscosity | 300-700 @ 10.0 wt% |
| Satialgine^{™} S 20 NS | Low viscosity | 300-600 @ 4.0 wt% |
| Satialgine^{™} S 60 NS | Low viscosity | 20-50 |
| Protanal^{®} LF10/60FT | Low viscosity | 30-60 |
| Manucol^{®} DH MCLDHP | | 40-90 |
| Manucol^{®} LKX50DR | Medium viscosity | 60-170 |
| Satialgine^{™} S 1100 | | 550-750 |
| Protanal^{®} CR 8223 | | 600-900 @ 1.25 wt%. |
| Satialgine^{™} S 1600 NS | High viscosity | 800-1000 |
| Protanal^{®} PH 6160 | High viscosity | 1000-1500 |
| Protanal^{®} PH 6160 | High viscosity | 1000-1500 |

The table 1 above provides a partial list of alginate products representing viscosity grades. Apparent viscosity is generally assayed at specified alginate weight percent in aqueous solution, measured at 20°C on a Brookfield RV viscometer, at 20 rpm, using spindle #2.

The poorly soluble bioactive material may be CoQ10. The CoQ10 utilized in the film of the invention is CoQ10 and its derivatives and analogue compounds is of the structural formula: 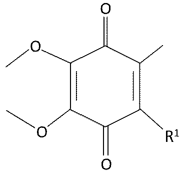 wherein R¹ is selected from the groups of:
1) a chain of 6-10 isoprenyl groups: 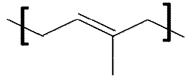 ; or
2) a linear or branched alkyl having C₅ to C₁₅ carbon chain. The carbon chain may be substituted with alkyl groups having C₁-C₆ carbon chain, hydroxyl group(s), or modified with cationic moieties, *inter alia,* triphenyl phosphonium group(s).

Other poorly soluble bioactive materials may include cannabinoids. The cannabinoids of the invention are pure or crude or semi-purified plant extracts, or synthetic or semi-synthetic cannabinoids, or cannabinoids analogs such as CBD or THC, or endocannabinoids, such as anandamide, or blend of two or more cannabinoids. The poorly soluble vitamins utilized in the film of the invention may be selected from the group consisting of vitamin A, vitamin D vitamin E, and vitamin K. Plant extracts may be selected for example from the group of delphinol, pycogenol, saffron, lutein, aragan oil. Examples of antifungal agents include clotrimazole, econazol, and lanconazole. Example of terpenes encompass both terpenes and terpenoids, and geraniol, citral, menthol, myrcene, and limonene. Specific examples of phytosterols include cyclic alcohols such as β-sitosterol, stigmasterol, brassicasterol and campesterol. Specific examples of flavonoids include tiliroside, quercetin, kaempferol, rutin, luteolin and isoflavones. Specific examples of poorly soluble phenols include caffeic acid, sesamol, curcumin, and ellagic acid.

The film may further comprise additional excipients, e.g. plasticizers, sweeteners, fillers (bulking agents), co-film-formers, stabilizers, including antioxidants, preservatives, buffering agents, chelating agents, flavors, colorants and the like.

Plasticizers are materials, as known in the art that impart flexibility to the film. The plasticizers suitable for the purposes of the present invention are usually compatible with alginates. The suitable plasticizers include polyols, e.g. sucrose, mannitol, sorbitol, xylitol, polyethylene glycol (PEG), e.g. PEG 400, and glycerol. The particularly preferred plasticizer is xylitol.

The plasticizers, particularly sugars, may also provide palatable taste to the film. However, additional natural or synthetic intensive sweeteners may be used including sucralose, stevia, aspartame, potassium acesulfame, and naringin dihydrochalcone.

The films may further comprise fillers, i.e. bulking agents, as known in the art. The purpose of bulking agents in the film may be to assist in disintegration of the film, or to increase the solid content of the active mass. These include microcrystalline cellulose, and insoluble fibers.

The films may further comprise additional film formers (co-film formers), e.g. to assist the formation of the alginate film or to improve its mechanical or organoleptic properties. The suitable additional film forming polymers include maltodextrins, pullulan, and soluble cellulose derivatives, e.g. hypromellose (hydroxypropyl methyl cellulose: HPMC), hydroxypropyl cellulose, and hydroxyethyl cellulose. Other co-film-formers include proteins such as gelatin, and starches or modified starches. The amount of none of these film formers in the film is larger than that of alginate. Preferably, the amount of all co-film-formers cumulatively is less than the amount of alginate in the film.

The films may further comprise stabilizers, e.g. to prevent degradation of the components of the film, or to prevent interaction thereof. Suitable preferred stabilizers include xanthan gum and carrageenan, and may also include gum arabic. Antioxidants may usually prevent oxidation by the atmospheric oxygen or metal impurities. Suitable preferred antioxidants include ascorbyl palmitate and tocopherols, and may also include conventional pharmaceutical antioxidants, e.g. ascorbic acid, thiols, or bisulfite. Preservatives may usually prevent or slow down microbial growth in the film. Suitable preservatives include sorbates or benzoates. Buffering agents usually assist in maintaining the pH value of the film. Suitable preferred buffering agents include citric acid and citrate salts, e.g. sodium citrate, and may also include phosphate, or acetate salts. Chelating agents may be used to decrease the free concentration of certain metals, including calcium, which may take part in chemical interactions with film's components, and may be a necessary microbial element. Preferred chelating agents include ethylenediamine tetraacetic acid (EDTA) and its salts, e.g. sodium salts, and may also include amino acids. Suitable emulsifiers are generally described above, but the films may comprise a combination of emulsifiers, and a combination of emulsifiers and co-emulsifiers, as known in the art.

The films may further comprise flavors and colorants, to improve palatability and appeal of the product. Suitable preferred flavors include plant extracts including oils and oleoresins, in particular may include mint extracts, and citrus oils. Generally, the term "flavors" as used herein refers to a group of chemical agents or molecules which may alter the flavor of another substance. The flavors may be of natural origin or a synthetic one, and may be related to the following groups: esters such as isoamyl acetate and ethyl propionate, terpenes such as menthol and limonene, alkamides such as spilanthol, amino acids or peptides such as glutamic acid, synthetic or natural compounds combined together to either imitate or enhance or mask a natural flavor, and mixture of natural and synthetic compounds. Suitable preferred colorants include chlorophyll, E110 (disodium 6-hydroxy-5-[(4-sulfophenyl)azo]-2-naphthalenesulfonate, also known as Sunset Yellow), and E132 (5,5'-indigodisulfonic acid sodium salt, known also as indigo carmine and blue FD&C#2).

The films may further comprise minerals, such as titanium dioxide to provide opacity or color.

**In** some further embodiments, the film of the invention may further comprise water soluble bioactive materials selected from the group consisting of vitamins, antioxidants, minerals, triptans, micronutrients, and homeopathic remedies. The concentration of the additional bioactive material in the final film may range between 0.001 %wt to 20 %wt.

The film of the invention may further be characterized by an oral residence time of between about one minute and about 90 minutes, more preferably between about 5 minutes and 30 minutes. Furthermore, the film may usually have an acceptable color and flavor, and a good mouth feel. The mouth feel may be as judged by an organoleptic panel evaluation, either professional or amateur. Furthermore, the film of the invention usually has a good adhesion to the roof of the mouth, and it is flexible, non-tacky and non-oily upon handling. The films of the invention may be applied to a variety of oral locations. The orally administrable mucoadhesive film may be at any place in the oral cavity, most preferably the roof of the mouth, or under the tongue.

The films according to the invention usually exhibit acceptable shelf life. That is, the films remain only minimally changed mechanically, physically or chemically, preferably for a period of at least two years upon storage under suitable conditions, e.g. at controlled room temperature (25 °C, with range of 15-30 °C). Recommended storage is in a dark place at ambient temperature in un-opened hermetically sealed packaging. In such storage the product is fit for use, i.e., no substantial chemical or physical degradation nor microbial spoilage take place which may prevent safe and effective use of the product. As demonstrated in the appended examples, the films retained physical appearance and mechanical stability upon storage for prolonged intervals.

### Unit dose

The area, length and width of the filmstrips of the invention is dictated by the required dose and the drug loading in the film. The area of the unit dose may vary between one square centimeter to twenty five square centimeters, e.g. between about 1x1 cm to about 5x5 cm. Preferably, the area of the unit dose of the film is between about 6 to about 12 square centimeters. A preferred length to width ratio is between 1:1 and 3:1, e.g. between 1.5:1 and 2.5:2. According to some embodiments, the dosage of the bioactive material in a single filmstrip may range from about 10 mg to about 200 mg. In some currently preferred embodiments, the bioactive material dosage is between about 40 mg to about 160 mg bioactive material, and more preferably between about 60 mg to about 100 mg bioactive material.

The term "chemically stable film" is interchangeable with the term 'stable film' as appears herein or in the claims refers to a film which retains its chemical-physical integrity, i.e. does not leach, change its composition or appearance or disintegrate upon storage under suitable conditions of storage, e.g., packed in sealed aluminum foil laminate, at ambient temperature, and displays a shelf-life of at least two year. The chemical stability of the film of the invention is defined such that not more than 10% of the original active ingredient concentration in the film is being degraded during appropriate storage as described above.

The term "about" as appears herein and in the claims refers to target set point of ±10% of the value which the term is related to.

### EXAMPLES

### Folding endurance

Folding endurance measurements as used herein can often exemplify and represent the mechanical strength and elasticity of a film. It was measured by folding a film at the same point several times until the film breaks. Strips of 8x2 cm of the film were used, each sample was bent upwards to form a 90° angle and then downwards to reach a 90° angle again in the opposite side. The score was assigned as follows: 1 - broken during one bending; 2 - cracked when bent 2 times; 3 - cracked when bent 3 times; 4 - cracked after bending 5 times; 5 - no crack after 10 iterations.

### Oral residence time

Oral residence time was evaluated in volunteers by cutting the films to strips, placement and adherence of the strips at the roof of the mouth of the volunteers, and measuring length of time until the physical presence of the formed swollen film is no longer detectable.

### Thickness of dry film

Measurements were performed with hand-held electronic micrometer, manufactured by MOTOKO, model MT 211101, with readout accuracy of ±1 micron.

### Viscosity

Unless specified otherwise, the measurements were performed with Brookfield -AMETEK DV2T viscometer. Detailed conditions of the measurements are described herein below.

### Moisture content

The film was incubated in the form of strips of 8x2 cm in size at 25 °C, and the relative humidity was recorded. The samples were weighed before incubation and every 24 hours until steady weight is reached. The samples were dried at 110 °C for 4 hours and stored for 24 hours in a desiccator over cuprum sulfate, and weighted again to measure moisture content.

### pH measurements

Measurements were performed with HANNA Digital pH meter model HI2211.

### Equipment

For high-shear homogenizing the following equipment was used: an overhead lab stirrer-type DLS (VELP Scientifica) with shear impeller - a blade disk of 45 mm diameter with 10 vertical blades, each at 5 mm width and 10 mm height at 20 degree from the shaft, or ultra turrax by DAIHAN Homogenizer HG-15A with dispersing tool Rotor Ø20mm and Stator Ø25mm, allowable maximal speed of 15,000 rpm. All other equipment used is described in the examples below.

All materials used in the examples were supplied by the sourced indicated therein. The lecithin that was used in all examples was liquid sunflower lecithin (E-322) Giralec (Lasenor Emul S.L., Spain).

### Example 1 - CoQ10 film preparation

Detailed amounts of the materials utilized are summarized in Table 2 herein below.

**Table 2**

| Ingredients | Amount, g | % wt in dry film. |
|---|---|---|
| Deionized water (pH 5.5) | 540 | |
| Sodium alginate Stialgine^{®} S20NS (*Algaia*) | 48 | 34.04 |
| Sodium alginate Stialgine^{®} S60NS (*Algaia*) | 12 | 8.51 |
| Coenzyme Q10, ubiquinone, *KANEKA* | 32 | 22.70 |
| Xylitol - Xylisorb 300, *Roquette* | 48 | 34.04 |
| Sunflower Lecithin | 0.1 | 0.07 |
| Stevia *- Intesse, SGF* | 0.36 | 0.26 |
| Tocopherol (*DSM*) | 0.01 | 0.01 |

CoQ10 powder (ubiquinone (manufactured by *KANEKA))* were melted in a glass beaker placed on a water bath, together with lecithin and tocopherol at 60°C.

In a separate container, sodium alginate (Satialgine S20NS and Satialgine S60NS (manufactured by *Algaia))* was dissolved in deionized water at 50°C. Stevia and xylitol were then added to the solution and dissolved at 50°C. The melted CoQ10 mixture was added to the alginate solution kept at 55°C and mixed using high shear impeller at 1500 rpm for 20 min until a homogeneous mixture was obtained. Thereafter, flavors were added and mixed uniformly under continuous heating to maintain 55°C.

The resulting active mass was cast onto a PET liner, 125 µm thick, (manufactured by DuPont) using a type draw blade with nominal gap of 1500 micron, and dried in a ventilated drying oven, at 80°C for 40 minutes.

### Results:

The obtained dry film had a thickness of 0.282±0.015 mm, and weight per area of 387 gr/m². Coenzyme Q10 content was 13.1 mg/cm². The film was tested for oral acceptance by placing on the tongue and adherence to the roof of the mouth. The film was found to have good flavor, good mouth feel and good adhesion to the roof of the mouth. Oral disintegration time was over 3 minutes. Upon handling the film was non-tacky, non-oily and flexible.

### Example 1a - comparative homogenization process

The ingredients used are presented in the table 3 below.

A premix of CoQ10 powder (ubiquinone (KANEKA)) together with lecithin and tocopherol was melted at 60°C (in a glass beaker placed in a water bath) was added slowly into the deionized water pre-heated to 55°C, and completely dispersed therein by Ultra turrax, applying one minute intervals shear-mixing at 20%, 30%, 40% and 50% of the maximum instrument speed, keeping the temperature between 54.5°C to 57.3°C.

Sodium alginate (Satialgine S20NS and Satialgine S60NS (Algaia)), Stevia and xylitol were uniformly mixed and added slowly to the CoQ10 emulsion and dissolved completely with flow turbo impeller at 1500 rpm for 25 minutes at 59°C. Flavors were added and afterwards air bubbles removed by stirring at 400 rpm for 15 minutes at 57°C.

**Table 3- Ingredients quantities**

| Ingredients | Amount (g) | % wt in dry film |
|---|---|---|
| Deionized water (pH 5.5) | 539.8 | |
| Sodium alginate Stialgine^{®} S20NS (*Algaia*) | 48.09 | 34.04 |
| Sodium alginate Stialgine^{®} S60NS (*Algaia*) | 12.37 | 8.51 |
| Coenzyme Q10, ubiquinone, *KANEKA* | 32.1 | 22.70 |
| Xylitol - Xylisorb 300, *Roquette* | 48.5 | 34.04 |
| Flavors | 0.6 | 0.42 |
| Sunflower Lecithin | 0.12 | 0.07 |
| Stevia *- Intesse, SGF* | 0.37 | 0.26 |
| Tocopherol (*DSM*) | 0.08 | 0.01 |

Viscosity of the formed active mass was 5,400 cPs (Spindle 4, 20 RPM, 57°C).

The resulting active mass was cast at 57°C onto a PET liner using a draw blade with nominal gap of 1500 micron and dried in a ventilated drying oven at 80°C for 45 minutes.

The same CoQ10: alginate ratio, CoQ10 percent in the dry film, casting of the active mass, and drying were used for this example and in the Example 1. Unlike the film formed by the method of Example 1, the formed film was dry, had several cracks and gaps, and was non-uniform.

Film had thickness was 0.167±0.010 mm, weights per area of 258 GSM. Coenzyme Q10 content was 5.8 mg/cm². The film had non-uniform orange color, and good flavor and mouth feel. Oral disintegration time was about 2 minutes, and folding endurance score was 5.

### Example 2 -film preparation, continuous drying process

CoQ10 powder (ubiquinone *(KANEKA))* were melted with lecithin and tocopherol at 60°C, in a glass beaker placed in a water bath. In a separate container, citric acid and tri-sodium citrate were added to deionized water until pH was obtained at 5.5. Sodium alginate (Satialgine S20 and Satialgine S60 *(Algaia))* was dissolved in the described aqueous solution at 50°C. Stevia, xylitol and maltodextrin were then added to the aqueous alginate solution and dissolved at 50°C. The melted CoQ10 mixture was added to the alginate solution at 55°C and mixed with high shear impeller at 1500 rpm. Flavors were then added. The amounts are presented in the table 4 below.

**Table 4**

| Ingredients | Amount, g | % wt in dry film. |
|---|---|---|
| Deionized water (pH 5.5) | 540 | |
| Sodium alginate Stialgine^{®} S20NS (Algaia) | 48 | 22.06 |
| Sodium alginate Stialgine^{®} S60NS (Algaia) | 12 | 5.52 |
| Coenzyme Q10, ubiquinone, KANEKA | 60 | 27.58 |
| Xylitol - Xylisorb 300, Roquette | 48 | 2.06 |
| Maltodextrin DE2-4 Paselli^{™} SA2 (Avebe) | 48 | 22.06 |
| Flavors | 0.6 | 0.28 |
| Sunflower Lecithin | 0.6 | 0.28 |
| Stevia - Intesse, SGF | 0.36 | 0.17 |
| Tocopherol (DSM) | 0.01 | 0.005 |

The obtained active mass was characterized by having a viscosity of 20,000 cP at 50°C (VEVOR Digital Rotary Viscometer model NDG 8S, spindle #4, 12 rpm). The casting and drying of said active mass was carried out at 50°C onto a PET release liner using a U-shaped Optimags MBCD 1.3-3.1 machine. The machine has 1600 mm drying length and 2 dryer zones. The web linear speed was 0.05 m/min, corresponding to about 32 minutes drying time. The temperature in the first zone was 70°C and 100°C in the second zone.

### Results:

The wet films thickness was 1275 µm upon drying under continuous operation, and wet film thicknesses over 1275 µm were dried in a drying oven.

The obtained dry film with moisture content about 12% had thickness of 307±0.015 mm, and weight per area of 409 gr/m², was tested for oral acceptance (taste, adhesion and residence time). The film was found to be flexible, uniform, to have good flavor, good mouth feel and good adhesion to the roof of the mouth. Oral disintegration time was 3-5 minutes.

Upon handling the film was non-tacky and non-oily and the folding endurance test score was 5.

Uniformity of the obtained film was calculated by weighing 7 samples of 1.9 cm x1.9 cm. The samples were taken from different parts of the film roll produced such that two samples were taken from each side, and three more samples were taken from the center of the obtained one meter length roll.

The weight per area ratio of the film samples was calculated by dividing the weight of the sample in the area (3.8 cm²) and calculated as grams per square meter (GSM).

The average weight was found to be 409 GSM, with a low standard deviation value (SD) of 4%. The SD achieved for the film achieved utilizing the process of the invention is significantly lower than that found in in other conventional film processes (SD about 10%).

The results are provided in Table 5:

| # | weight | GSM |
|---|---|---|
| 1 | 145.2 | 382 |
| 2 | 153.9 | 405 |
| 3 | 155.8 | 410 |
| 4 | 159.5 | 420 |
| 5 | 166.5 | 438 |
| 6 | 157.5 | 414 |
| 7 | 149.3 | 393 |
| | Average | 409 |
| | SD | 16 (4%) |

Moisture content was determined by loss on drying to be between 11 % to 15 %wt.

The film was cut to units of 2.5 cm by 3.2 cm (area 8 cm²). The units' average weight was about 327 mg, and average calculated content of about 100 mg CoQ10 per unit.

### Example 3 - Grinding formulation

Detailed amounts of the materials utilized are summarized in Table 6 herein below.

**Table 6**

| Ingredients | Amount, g | % wt in dry film |
|---|---|---|
| Deionized water (pH 5.5) | 540 | |
| Sodium alginate Stialgine^{®} S20NS (*Algaia*) | 48 | 34.03 |
| Sodium alginate Stialgine^{®} S60NS (*Algaia*) | 12 | 8.51 |
| Coenzyme Q10, ubiquinone, *KANEKA* | 32 | 22.68 |
| Xylitol - Xylisorb 300, *Roquette* | 48 | 34.03 |
| Flavors | 0.6 | 0.43 |
| Sunflower Lecithin | 0.1 | 0.07 |
| Stevia *- Intesse, SGF* | 0.36 | 0.26 |
| Tocopherol (*DSM*) | 0.01 | 0.01 |

CoQ10 (ubiquinone *(KANEKA))* powder were melted with lecithin and tocopherol at 60°C. The molten mixture was then poured into an electric high-speed rotating knife grinding machine containing xylitol for about three minutes, in short intervals, to maintain the temperature below 40°C. Obtained particles were passed through 60 mesh sieve.

In a separate vessel, sodium alginate (Satialgine S20NS and Satialgine S60NS *(Algaia))* was dissolved in deionized water at 50°C. Stevia was then added to the solution and dissolved at 50°C.

The powder of the finely ground mixture was then added to the alginate solution at 55°C and mixed with a high shear impeller at 1500rpm for 20 min, afterwards flavors were added and mixed.

The resulting active mass was cast onto liner using a draw blade with nominal gap of 1500 micron, and dried in a ventilated drying oven, at 80°C for 40 minutes.

### Results:

The obtained dry film had a thickness of 0.228±0.030 mm, and weight per area of 312 gr/m². Coenzyme Q10 content was 7.0 mg/cm². The film was tested for oral acceptance (taste, adhesion and residence time). The film was found to have good flavor, good mouth feel and good adhesion to the roof of the mouth. Oral disintegration time was about 3 to 5 minutes. Upon handling the film was non-tacky and non-oily.

### Example 4 - melting CoQ10 in the absence of additional ingredients

This formulation was carried out by incorporating the lecithin component into the aqueous film forming polymer solution, while the CoQ10 was melted at 60°C with no additional ingredients. The melted CoQ10 was added to the aqueous solution as described in the former example.

The formed active mass was then cast with draw blade at 1,000 micron gap, and dried at 90°C.

**Table 7**

| Ingredients | Amount, g | % wt in dry film |
|---|---|---|
| Deionized water (pH 5.5) | 210 | |
| Sodium citrate | 1.65 | 1.39 |
| Citric acid | 0.3 | 0.25 |
| Sodium alginate Stialgine^{®} S20NS (*Algaia*) | 18 | 15.11 |
| Sodium alginate Protanal^{®} LFR 5/60 (*DuPont*) | 18 | 15.11 |
| Xylitol - Xylisorb 300, *Roquette* | 45 | 37.77 |
| Coenzyme Q10, ubiquinone, *KANEKA* | 18 | 15.11 |
| Maltodextrin DE2-4 Paselli^{™} SA2 (*Avebe*) | 18 | 15.11 |
| Sunflower Lecithin | 0.18 | 0.15 |

### Results:

The formed film thickness 0.300±0.015 mm, having folding endurance score of 4. The film's surface was non oily and non-tacky.

### Example 5 - CoQ10 high loading

Detailed amounts of the materials utilized are summarized in Table 8 herein below.

CoQ10 (ubiquinone *(KANEKA))* powder were melted with lecithin and tocopherol at 60°C.

In a separate container, sodium alginate (Satialgine S20NS and Satialgine S60NS (manufactured by *Algaia*)) was dissolved in deionized water at 50°C. Stevia and xylitol were then added to the solution and dissolved at 50°C. The melted CoQ10 mixture was added to the alginate solution kept at 55°C and mixed using high shear impeller at 1500 rpm for 20 min until a homogeneous mixture was obtained. Thereafter, flavors were added and mixed uniformly under continuous heating to maintain 55°C.

**Table 8**

| Ingredients | Amount, g | % wt in dry film |
|---|---|---|
| Deionized water (pH 5.5) | 124.73 | |
| Sodium citrate | 0.99 | 1.19 |
| Citric acid | 0.19 | 0.23 |
| Sodium alginate Stialgine^{®} S20NS (*Algaia*) | 13.77 | 16.53 |
| Sodium alginate Stialgine^{®} S60NS (*Algaia*) | 0.86 | 1.03 |
| Coenzyme Q10, ubiquinone, *KANEKA* | 36.57 | 43.92 |
| Xylitol - Xylisorb 300, *Roquette* | 15.49 | 18.60 |
| Maltodextrin DE2-4 Paselli^{™} SA2 (*Avebe*) | 10.33 | 12.40 |
| Flavors | 1.17 | 1.40 |
| Sunflower Lecithin | 3.82 | 4.59 |
| Stevia *- Intesse, SGF* | 0.04 | 0.05 |
| Tocopherol (*DSM*) | 0.04 | 0.05 |

The resulting active mass was cast onto liner using a draw blade with nominal gap of 1000 micron, and dried in a ventilated drying oven, at 110°C for 12 minutes.

### Results:

The obtained dry film had a thickness of 0.402±0.038 mm, and weight per area of 250 gr/m². Coenzyme Q10 content was 10.9 mg/cm². The film was tested for oral acceptance (taste, adhesion and residence time). The film was found to have good flavor, good mouth feel and good adhesion to the roof of the mouth. Folding endurance score of 5. Upon handling the film was non-tacky and non-oily.

### Example 6 - Omega 3 oil

The composition of the formulation and the amounts of the ingredients are shown in the table 9 below.

Sodium alginate (Satialgine S20NS and Satialgine S60NS (Algaia)) was dissolved in deionized water with Xylitol and maltodextrin at 50°C. The alginate solution was added to the lecithin a uniform solution at 50°C. Omega 3 Oil (BASF) was added to the solution at 50°C and mixed with a high shear impeller at 1500 rpm for 20 min, until a uniform solution was obtained.

The resulting active mass was cast onto a liner using a draw blade with nominal gap of 1000 micron, and dried in a ventilated drying oven, at 110°C for 12 minutes.

**Table 9 - film content**

| Ingredients | Amount, g | % wt in dry film. |
|---|---|---|
| Deionized water (pH 5.5) | 146 | |
| Tri sodium citrate | 1.08 | 1.50 |
| Citric acid | 0.21 | 0.29 |
| Sodium alginate Stialgine^{®} S20NS (Algaia) | 16.62 | 23.11 |
| Sodium alginate Stialgine^{®} S60NS (Algaia) | 1.04 | 1.44 |
| Xylitol - Xylisorb 300, Roquette | 18.7 | 26.00 |
| Maltodextrin DE2-4 Paselli^{™} SA2 (Avebe) | 12.47 | 17.34 |
| Omega 3 (BASF) | 19.62 | 27.28 |
| Sunflower Lecithin | 2.18 | 3.03 |

The obtained dry film had thickness of 0.246±0.014 mm, and weight per area of 410 GSM. Omega 3 content was 11.1 mg/cm². Folding endurance score was 5. The oral acceptance (adhesion and residence time) was typical and good, good mouth feel and good adhesion to the roof of the mouth. The film was of light color with a smooth texture. Upon handling, the film was non-tacky and had slight oil fluting that reduced over time.

### Example 7 - Natural vitamin E

The composition of the formulation and the amounts of the ingredients are shown in the table 10 below.

Sodium alginate (Satialgine S20NS and Satialgine S60NS *(Algaia))* was dissolved in deionized water with Xylitol and maltodextrin at 50°C. The alginate solution was added to the lecithin and to a uniform solution at 50°C. Natural vitamin E *(DSM)* was added to the solution at 50°C and mixed with high shear impeller at 1500 rpm for 20 min, until a uniform solution was obtained.

The resulting active mass was cast onto a liner using a draw blade with nominal gap of 1000micron, and dried in a ventilated drying oven, at 110°C for 12 minutes.

**Table 9 - film content**

| Ingredients | Amount, g | % wt in dry film. |
|---|---|---|
| Deionized water (pH 5.5) | 146.75 | |
| Tri sodium citrate | 1.08 | 1.50 |
| Citric acid | 0.21 | 0.29 |
| Sodium alginate Stialgine^{®} S20NS (Algaia) | 16.69 | 23.11 |
| Sodium alginate Stialgine^{®} S60NS (Algaia) | 1.04 | 1.44 |
| Xylitol - Xylisorb 300, Roquette | 18.78 | 26.00 |
| Maltodextrin DE2-4 Paselli^{™} SA2 (Avebe) | 12.52 | 17.34 |
| Natural vitamin E (DSM) | 19.71 | 27.28 |
| Sunflower Lecithin | 2.19 | 3.03 |

The obtained dry film had thickness of 0.234±0.010 mm, and weight per area of 343 GSM. Natural Vitamin E content was 9.3 mg/cm². Folding endurance score was 5. The oral acceptance (adhesion and residence time) was typical and good, good mouth feel and good adhesion to the roof of the mouth. The film was of light color with a smooth texture. Upon handling, the film was non-tacky.

### Example 8 - Curcuma Longa Loading 1:1

The composition of the formulation and the amounts of the ingredients are shown in table 11 below.

Sodium alginate (Satialgine S20NS and Satialgine S60NS *(Algaia))* was dissolved in deionized water with Xylitol and maltodextrin at 50°C. The curcuma longa powder *(Bara,* Israel, lot: CLP1911419) was added to the solution with lecithin and mixed with a high shear impeller at 1500 rpm for 20 min at 50°C until a uniform solution was obtained.

The resulting active mass was cast onto a liner using a draw blade with nominal gap of 1000 micron, and dried in a ventilated drying oven, at 110°C and 60°C for 14 minutes and 20 minutes, respectively.

**Table 11 - film content**

| Ingredients | Amount, g | % wt in dry film. |
|---|---|---|
| Deionized water (pH 5.5) | 147.55 | |
| Tri sodium citrate | 1.09 | 1.55 |
| Citric acid | 0.21 | 0.30 |
| Sodium alginate Stialgine^{®} S20NS (*Algaia*) | 16.79 | 23.84 |
| Sodium alginate Stialgine^{®} S60NS (*Algaia*) | 1.05 | 1.49 |
| Xylitol *- Xylisorb* 300, *Roquette* | 18.89 | 26.82 |
| Maltodextrin DE2-4 Paselli^{™} SA2 (*Avebe*) | 12.59 | 17.88 |
| Curcuma longa 95% water-insoluble extract 1:15 (*BARA,* Israel, lot: CLP1911419) | 19.62 | 27.85 |
| Sunflower Lecithin | 0.19 | 0.28 |

The film characteristics were the same in both drying temperatures.

The obtained dry film had thickness of 0.296±0.017 mm, and weight per area of 410 GSM. Curcuma longa content was 11.4 mg/cm². Folding endurance score was 5. The oral acceptance (taste, adhesion and residence time) was typical and good, pleasant taste, good mouth feel and good adhesion to the roof of the mouth. The film was non-tacky.

The film was of orange color and there were few particles of curcuma longa that did not dispersed well in the solution.

### Example 9 - Lecithin Loading 2.5:1

The composition of the formulation and the amounts of the ingredients are shown in table 12 below.

Sodium alginate (Satialgine S20NS and Satialgine S60NS *(Algaia))* was dissolved in deionized water with Xylitol and maltodextrin at 50°C. The alginate solution was added to the lecithin and mixed with a high shear impeller at 1500 rpm for 20 min at 50°C.

**Table 12 - film content**

| Ingredients | Amount, g | % wt in dry film. |
|---|---|---|
| Deionized water (pH 5.5) | 150.2 | |
| Tri sodium citrate | 1.11 | 1.11 |
| Citric acid | 0.21 | 0.21 |
| Sodium alginate Stialgine^{®} S20NS (*Algaia*) | 17.09 | 17.13 |
| Sodium alginate Stialgine^{®} S60NS (*Algaia*) | 1.06 | 1.07 |
| Xylitol *- Xylisorb* 300, Roquette | 19.23 | 19.27 |
| Maltodextrin DE2-4 Paselli^{™} SA2 (*Avebe*) | 12.82 | 12.85 |
| Sunflower Lecithin | 48.25 | 48.36 |

The resulting active mass was cast onto a PET liner using a draw blade with nominal gap of 1000 micron, and dried in a ventilated drying oven, at 110°C for 22 minutes.

The obtained dry film had thickness of 0.573 ±0.381 mm, and weight per area of 739 GSM. Lecithin content was 11.1 mg/cm². Folding endurance test score was 5. The film was of light brown color and contained several aggregates of poorly dispersed powder. The film was non-tacky but exhibited slight oiliness.

### Example 10 - Lecithin as bioactive material, loading 1:1

The composition of the formulation and the amounts of the ingredients are shown in table 13 below.

Sodium alginate (Satialgine S20NS and Satialgine S60NS *(Algaia))* was dissolved in deionized water with xylitol and maltodextrin at 50°C. The alginate solution was added to the lecithin with a high shear impeller at 1500 rpm for 20 min at 50°C.

The resulting active mass was cast onto a PET liner using a draw blade with nominal gap of 1000 micron, and dried in a ventilated drying oven, at 110°C for 18 minutes.

**Table 13 - film content**

| Ingredients | Amount, g | % wt in dry film. |
|---|---|---|
| Deionized water (pH 5.5) | 148.77 | |
| Tri sodium citrate | 1.10 | 1.59 |
| Citric acid | 0.21 | 0.31 |
| Sodium alginate Stialgine^{®} S20NS (*Algaia*) | 16.93 | 24.49 |
| Sodium alginate Stialgine^{®} S60NS (*Algaia*) | 1.06 | 1.53 |
| Xylitol *- Xylisorb* 300, *Roquette* | 19.05 | 27.55 |
| Maltodextrin DE2-4 Paselli^{™} SA2 (*Avebe*) | 12.70 | 18.37 |
| Sunflower Lecithin | 18.09 | 26.16 |

The obtained dry film had thickness of 0.216 ±0.011 mm, and weight per area of 504 GSM. Lecithin content was 13.1 mg/cm². Folding endurance test score was 5. The film was of yellow color with small aggregates of poorly dispersed powder, considerably smaller than in example 8. The film was non-tacky but exhibited slight oiliness.

### Example 11 - Lutein, loading ratio 1:2

First, an alginate solution was prepared. Xylitol and Maltodextrin, and Alginate Protanal^{®} LFR 5/60 (FMC/Dupont) were dissolved in deionized water at ambient temperature (about 22°C), by mixing with high shear impeller at 1500 rpm for 20 min.

The viscosity of alginate solution was 2,700 cPs (Spindle #4, 20 rpm, at 22°C)

Thereafter, lutein (Lycored, Lyc-O-Lutein 20% in safflower oil and minor quantity of Rosemary Extract) and lecithin were added the alginate solution at room temperature and mixed with a high shear impeller at 1500 rpm for 20 min, until forming a unified active mass formulation.

**Table 14 - film content**

| Ingredients | Amount, g | % wt in dry film. |
|---|---|---|
| Deionized water (pH 5.5) | 143.73 | |
| Tri sodium citrate | 1.10 | 1.01 |
| Citric acid | 0.21 | 0.19 |
| Sodium alginate Protanal^{®} LFR 5/60 (DuPont) | 20.01 | 18.25 |
| Xylitol - Xylisorb 300, Roquette | 22.01 | 20.08 |
| Maltodextrin DE2-4 Paselli^{™} SA2 (Avebe) | 22.01 | 20.08 |
| Lutein (Lycored) | 40.26 | 36.73 |
| Sunflower Lecithin | 4.03 | 3.67 |

Viscosity of the active mass was 4,200 cPs (Spindle #4, 20 rpm, at 22°C).

The active mass was cast onto a PET liner using a draw blade with nominal gap of 500 micron, and dried in a ventilated drying oven at 90°C for 10 minutes.

The obtained dry film had thickness of 0.214±0.010 mm. Obtained film weight per area was 390 GSM. Lutein content was 14.3 mg/cm². Obtained film had somewhat weak adhesion to the roof of the mouth with residence time of about 90 seconds. Film was readily cut into dosage units, was flexible, with folding endurance score of 2. The flavor and mouth feel were mild and acceptable.

### Comparative example 1

The purpose of this trial is to perform comparative examples based on the Japanese patent JP 2018-203618 A.

The compositions of the prepared solutions are summarized in the Table 15 below.

Preparation 1 was a solution with 20% octenyl succinic anhydride (OSA) starch Preparation 2 was a solution with 20% OSA starch and xylitol (1: 1 ratio). Preparation 3 was the composition of the example 5 from the Japanese patent JP 2018-203618. Preparation 4 was the composition of the example 6 from the Japanese patent JP 2018-203618.

**Table 15**

| | Preparation #1 | Preparation #2 | Preparation #3 Example 5 | Preparation #4 Example 6 |
|---|---|---|---|---|
| Water (deionized) | 160 gr | 160gr | 100 gr | 100 |
| OSA starch (CLEARGUM CO03 Roquette) | 40 gr | 40gr | 20 | 40 |
| Xylitol (Xylisorb 300, Roquette) | | 40gr | - | |
| CoQ10 (KANEKA) | | | 20 | 40 |
| Na Alginate LFR (FMC) | | | 60 | 20 |

OSA starch was weighed according to the table above, and added into the purified water under mixing. The pH was adjusted to 5-8 using tri-sodium citrate / citric acid. The mixture was heated to 60°C and mixed until dissolution. CoQ10 was then added into solution in 60°C, and homogenized by using ultra turrax applying one minute intervals shear-mixing at 40% of the maximum instrument speed for a total of 2 min. Alginate LFR was then added and mixed well at 60°C, using high shear impeller. The resultant solutions were cast on a PET release liner with 1000 micron gap. Drying was performed at 110°C in a static oven with ventilation. The dry films were evaluated for GSM, and folding endurance. Preparations #1 and #2 produced low viscosity solutions which could not be cast properly. The obtained residues, however, were in a form of brittle film. This indicates that OSA-starch is a film-forming polymer.

Preparation #3 was readily prepared. Dissolving of OSA starch at 60°C in water was easy, with some foaming. The addition of CoQ10 to the solution was also smooth and the solution was homogenized easily with ultra turax. The addition of alginate to the emulsion created a very viscous solution which could not be mixed with the high shear impeller. Therefore, water was added to the solution - addition of 200 gr of water was required in order to reduce the viscosity and allow proper mixing. Casting was done on a PET release liner, with 1000 micron gap. Drying was done at 110°C (modified procedure) and at 60°C (original procedure). Film obtained from drying at 110°C separated from the liner and multiple cracks were formed. Film obtained from drying at 60°C demonstrated cracks, but than in the 110°C. In both films it was not possible to measure GSM, nor folding endurance. The films were brittle and could not be handled.

Preparation #4 was then modified from the start with additional 100 gr of water (total 200 gr) to avoid high viscosity observed in the preparation 3. All the stages of process as described in previous preparations were good and easily achieved emulsion and mixing. Casting was done on a release liner, 1000-micron gap. Drying was done at 110°C and at 60°C. Drying at 60 °C required significantly prolonged time - 36 minutes. The obtained films detached from the liner and formed significant cracks. The mechanical properties of both films were very poor so it was not possible to measure GSM nor folding endurance.

## Claims

1. A method of manufacturing of an orally administrable mucoadhesive film, in which alginate is the major film-forming polymer, comprising a step of:
homogenizing an aqueous solution comprising one or more alginates, with at least one poorly water-soluble bioactive material and at least one emulsifier, to provide a mass suitable for casting,
wherein the homogenization is achieved under high weight ratio of the bioactive material(s) to the alginate(s) wherein said high weight ratio between said bioactive material and said alginate is from 1:2.5 to 2.5:1, and wherein the homogenization is performed using high-shear mixers selected from rotor-stator type homogenizers or mechanical mixers equipped with high-shear impeller.

2. The method according to claim 1, comprising, prior to homogenizing, the steps of:
1a) combining the poorly water-soluble bioactive material and the emulsifier, and
1b) combining a mixture resultant from step 1a) with the alginate solution; or
2a) combining the alginate solution with said emulsifier, and
2b) combining a mixture resultant from step 2a) with the poorly water-soluble bioactive material; or
3a) mixing said poorly water-soluble bioactive material and an emulsifier,
3b) combining a mixture resultant from step 3a) with an adsorbing powder,
3c) grinding a mixture obtained at step 3b), and
3d) combining a mixture of step 3c) with the alginate solution.

3. The method according to any one of preceding claims, wherein the poorly water-soluble bioactive material is coenzyme Q10.

4. The method according to any one of claim 1 to 3, wherein the amount of the emulsifier is lower than 1/10 of the amount of the bioactive material in the film.

5. The method according to any one of claims 1 to 4, wherein the emulsifier is lecithin.

6. The method according to any one of claims 1 to 5, further comprising casting said active mass onto a liner at a wet thickness between 1,250 and 3,000 micrometers, and drying the film.

7. The method according to claim 6, wherein said casting is performed by a slit-die apparatus at a temperature between 50°C and 65°C, and the drying of the film is performed at a temperature between 90°C and 110°C.

8. The method according to any one of the preceding claims, wherein the weight ratio between the bioactive material and the alginate is from 1:1-2.5:1.

9. An orally administrable mucoadhesive film comprising at least one poorly water-soluble bioactive material, at least one emulsifier, and an alginate as major film forming polymer, wherein the concentration of said poorly water-soluble bioactive material in the film is between 15 wt% and 45 wt% of the dry weight of said film, wherein the film comprises between 5 and 20 milligrams of the poorly water-soluble bioactive material in a square centimeter of the film with dry film thickness varying in the range from 100 to 400 µm, and wherein the weight ratio between the bioactive material and the alginate is from 1:2.5 to 2.5:1.

10. The film according to claim 9, wherein the weight ratio between said bioactive material and said alginate is from 1:1 to 2.5:1.

11. The film according to any one of claims 9 or 10, wherein the concentration of the emulsifier is lower than 1/10 of the concentration of the bioactive material in said film.

12. The film according to claim 11, wherein the emulsifier is a zwitterionic emulsifier.

13. The film according to any one of claims 9 to 12, wherein the poorly water-soluble bioactive material is selected from the group consisting of vitamins and vitamin-like substances that are naturally present in the human body, carotenes, terpenes, flavonoids, cannabinoids, natural oils, and plant extracts, and wherein the film further comprises one or more stabilizers, antioxidants, buffering agents, and co-emulsifiers.

14. The film according to any one of claims 9 to 13, wherein said poorly water-soluble bioactive material has an aqueous solubility below 10 mg/L at 20 °C.

15. The film according to any one of claims 9 to 14, comprising from 15 to 45 wt% based on the dry weight of the film of coenzyme Q10 as the bioactive material, and the emulsifier is lecithin.

16. The film according to any one of claims 9 to 15, wherein film uniformity expressed in percentage, calculated by weighing units of the film normalized by their area, to obtain the mean and standard deviation of the weights, and dividing the standard deviation by the mean to give the uniformity, is lower than 12%.

## Patentansprüche

1. Verfahren zur Herstellung eines oral verabreichbaren mukoadhäsiven Films, in dem Alginat das hauptsächliche filmbildende Polymer ist, umfassend einen Schritt des:
Homogenisierens einer wässrigen Lösung, die ein oder mehrere Alginate enthält, mit mindestens einem schwer wasserlöslichen bioaktiven Material und mindestens einem Emulgator, um eine zum Gießen geeignete Masse bereitzustellen,
wobei die Homogenisierung unter einem hohen Gewichtsverhältnis des/der bioaktiven Materials/Materialien zu dem/den Alginat/Alginaten erreicht wird, wobei das hohe Gewichtsverhältnis zwischen dem bioaktiven Material und dem Alginat 1:2,5 bis 2,5:1 beträgt, und wobei die Homogenisierung unter Verwendung von Hochschermischern, ausgewählt aus Homogenisatoren vom Rotor-Stator-Typ oder mechanischen Mischern, die mit einem Hochscher-Impeller ausgestattet sind, durchgeführt wird.

2. Verfahren nach Anspruch 1, das vor dem Homogenisieren die folgenden Schritte umfasst:
1a) Kombinieren des schwer wasserlöslichen bioaktiven Materials und des Emulgators, und
1b) Kombinieren einer aus Schritt 1a) resultierenden Mischung mit der Alginatlösung; oder
2a) Kombinieren der Alginatlösung mit dem Emulgator, und
2b) Kombinieren einer aus Schritt 2a) resultierenden Mischung mit dem schwer wasserlöslichen bioaktiven Material; oder
3a) Mischen des schwer wasserlöslichen bioaktiven Materials und eines Emulgators,
3b) Kombinieren einer aus Schritt 3a) resultierenden Mischung mit einem adsorbierenden Pulver,
3c) Zerkleinern einer in Schritt 3b) erhaltenen Mischung, und
3d) Kombinieren einer Mischung aus Schritt 3c) mit der Alginatlösung.

3. Verfahren nach einem der vorhergehenden Ansprüche, wobei das schwer wasserlösliche bioaktive Material Coenzym Q10 ist.

4. Verfahren nach einem der Ansprüche 1 bis 3, wobei die Menge des Emulgators weniger als 1/10 der Menge des bioaktiven Materials in dem Film beträgt.

5. Verfahren nach einem der Ansprüche 1 bis 4, wobei der Emulgator Lecithin ist.

6. Verfahren nach einem der Ansprüche 1 bis 5, ferner umfassend das Gießen der aktiven Masse auf einen Liner mit einer Nassschichtdicke zwischen 1.250 und 3.000 Mikrometern und das Trocknen des Films.

7. Verfahren nach Anspruch 6, wobei das Gießen mit einer Schlitzdüsenvorrichtung bei einer Temperatur zwischen 50°C und 65°C durchgeführt wird und das Trocknen des Films bei einer Temperatur zwischen 90°C und 110°C durchgeführt wird.

8. Verfahren nach einem der vorhergehenden Ansprüche, wobei das Gewichtsverhältnis zwischen dem bioaktiven Material und dem Alginat 1:1-2,5:1 beträgt.

9. Oral verabreichbarer mukoadhäsiver Film, umfassend mindestens ein schwer wasserlösliches bioaktives Material, mindestens einen Emulgator und ein Alginat als hauptsächliches filmbildendes Polymer, wobei die Konzentration des schwer wasserlöslichen bioaktiven Materials in dem Film zwischen 15 Gew.-% und 45 Gew.-% des Trockengewichts des Films beträgt, wobei der Film zwischen 5 und 20 Milligramm des schwer wasserlöslichen bioaktiven Materials in einem Quadratzentimeter des Films umfasst, wobei die Trockenfilmdicke im Bereich von 100 bis 400 µm variiert, und wobei das Gewichtsverhältnis zwischen dem bioaktiven Material und dem Alginat 1:2,5 bis 2,5:1 beträgt.

10. Film nach Anspruch 9, wobei das Gewichtsverhältnis zwischen dem bioaktiven Material und dem Alginat von 1:1 bis 2,5:1 beträgt.

11. Film nach einem der Ansprüche 9 oder 10, wobei die Konzentration des Emulgators weniger als 1/10 der Konzentration des bioaktiven Materials in dem Film beträgt.

12. Film nach Anspruch 11, wobei der Emulgator ein zwitterionischer Emulgator ist.

13. Film nach einem der Ansprüche 9 bis 12, wobei das schwer wasserlösliche bioaktive Material aus der Gruppe bestehend aus Vitaminen und vitaminähnlichen Substanzen, die im menschlichen Körper natürlich vorkommen, Carotinen, Terpenen, Flavonoiden, Cannabinoiden, natürlichen Ölen und Pflanzenextrakten ausgewählt ist und wobei der Film ferner einen oder mehrere Stabilisatoren, Antioxidantien, Puffersubstanzen und Co-Emulgatoren umfasst.

14. Film nach einem der Ansprüche 9 bis 13, wobei das schwer wasserlösliche bioaktive Material eine Wasserlöslichkeit von unter 10 mg/L bei 20°C aufweist.

15. Film nach einem der Ansprüche 9 bis 14, der 15 bis 45 Gew.-%, bezogen auf das Trockengewicht des Films, Coenzym Q10 als bioaktives Material enthält und wobei der Emulgator Lecithin ist.

16. Film nach einem der Ansprüche 9 bis 15, wobei die Gleichmäßigkeit des Films, ausgedrückt in Prozent, berechnet durch Wiegen von auf ihre Fläche normierten Einheiten des Films, um den Mittelwert und die Standardabweichung der Gewichte zu erhalten, und Teilen der Standardabweichung durch den Mittelwert, um die Gleichmäßigkeit zu ergeben, weniger als 12% beträgt.

## Revendications

1. Procédé de fabrication d'un film mucoadhésif administrable par voie orale, dans lequel de l'alginate est le polymère filmogène principal, comportant une étape consistant à :
homogénéiser une solution aqueuse comportant un ou plusieurs alginates, avec au moins une matière bioactive faiblement soluble dans l'eau et au moins un émulsifiant, afin de fournir une masse adaptée pour la coulée,
dans lequel l'homogénéisation est obtenue sous un rapport pondéral élevé de la (des) matière(s) bioactive(s) sur l'alginate(s), dans lequel ledit rapport pondéral élevé entre ladite matière bioactive et ledit alginate est de 1:2,5 à 2,5:1, et dans lequel l'homogénéisation est réalisée en utilisant des mélangeurs à cisaillement élevé choisis parmi des homogénéisateurs de type rotor-stator ou des mélangeurs mécaniques équipés d'une hélice à cisaillement élevé.

2. Procédé selon la revendication 1, comportant, avant l'homogénéisation, les étapes consistant à :
1a) combiner la matière bioactive faiblement soluble dans l'eau et l'émulsifiant, et
1b) combiner un mélange résultant de l'étape 1a) avec la solution d'alginate ; ou
2a) combiner la solution d'alginate avec ledit émulsifiant, et
2b) combiner un mélange résultant de l'étape 2a) avec la matière bioactive faiblement soluble dans l'eau ; ou
3a) mélanger ladite matière bioactive faiblement soluble dans l'eau et un émulsifiant,
3b) combiner un mélange résultant de l'étape 3a) avec une poudre adsorbante,
3c) broyer un mélange obtenu à l'étape 3b), et
3d) combiner un mélange de l'étape 3c) avec la solution d'alginate.

3. Procédé selon l'une quelconque des revendications précédentes, dans lequel la matière bioactive faiblement soluble dans l'eau est la coenzyme Q10.

4. Procédé selon l'une quelconque des revendications 1 à 3, dans lequel la quantité de l'émulsifiant est inférieure à 1/10 de la quantité de la matière bioactive dans le film.

5. Procédé selon l'une quelconque des revendications 1 à 4, dans lequel l'émulsifiant est la lécithine.

6. Procédé selon l'une quelconque des revendications 1 à 5, comportant en outre de couler ladite masse active sur un revêtement à une épaisseur humide comprise entre 1 250 et 3 000 micromètres, et de sécher le film.

7. Procédé selon la revendication 6, dans lequel ladite coulée est réalisée par un appareil à filière plate à une température comprise entre 50 °C et 65 °C, et le séchage du film est réalisé à une température comprise entre 90 °C et 110 °C.

8. Procédé selon l'une quelconque des revendications précédentes, dans lequel le rapport pondéral entre la matière bioactive et l'alginate est de 1: 1 à 2,5: 1.

9. Film mucoadhésif administrable par voie orale comportant au moins une matière bioactive faiblement soluble dans l'eau, au moins un émulsifiant et un alginate en tant que polymère filmogène principal, dans lequel la concentration de ladite matière bioactive faiblement soluble dans l'eau dans le film est comprise entre 15 % en poids et 45 % en poids du poids à sec dudit film, dans lequel le film comporte entre 5 et 20 milligrammes de la matière bioactive faiblement soluble dans l'eau dans un centimètre carré du film avec une épaisseur de film sec variant dans la plage de 100 à 400 µm, et dans lequel le rapport pondéral entre la matière bioactive et l'alginate est compris de 1:2,5 à 2,5:1.

10. Film selon la revendication 9, dans lequel le rapport pondéral entre ladite matière bioactive et ledit alginate est de 1: 1 à 2,5: 1.

11. Film selon l'une quelconque des revendications 9 ou 10, dans lequel la concentration de l'émulsifiant est inférieure à 1/10 de la concentration de la matière bioactive dans ledit film.

12. Film selon la revendication 11, dans lequel l'émulsifiant est un émulsifiant zwitterionique.

13. Film selon l'une quelconque des revendications 9 à 12, dans lequel la matière bioactive faiblement soluble dans l'eau est choisie parmi le groupe constitué de vitamines et de substances analogues à des vitamines qui sont naturellement présentes dans le corps humain, des carotènes, des terpènes, des flavonoïdes, des cannabinoïdes, des huiles naturelles et des extraits de plantes, et dans lequel le film comporte en outre un ou plusieurs stabilisants, antioxydants, agents tampons et co-émulsifiants.

14. Film selon l'une quelconque des revendications 9 à 13, dans lequel ladite matière bioactive faiblement soluble dans l'eau a une solubilité aqueuse inférieure à 10 mg/l à 20 °C.

15. Film selon l'une quelconque des revendications 9 à 14, comportant de 15 à 45 % en poids, sur la base du poids à sec du film, de coenzyme Q10 en tant que matière bioactive, et l'émulsifiant est la lécithine.

16. Film selon l'une quelconque des revendications 9 à 15, dans lequel une uniformité de film exprimée en pourcentage, calculée en pesant des unités du film normalisées par leur surface, pour obtenir la moyenne et l'écart-type des poids, et en divisant l'écart-type par la moyenne pour obtenir l'uniformité, est inférieure à 12 %.
